# EUROPEAN PATENT SPECIFICATION

(11) **EP 2 815 712 B1**
(45) Date of publication and mention of the grant of the patent: **19.06.2019**
(21) Application number: 13749314.4
(22) Date of filing: 06.02.2013
(51) Int. Cl.: A61F 7/12

(54) **BRAIN COOLING DEVICE FOR OPEN-SKULL SURGERY PATIENT**
HIRNKÜHLUNGSVORRICHTUNG FÜR EINEN AM OFFENEN SCHÄDEL OPERIERTEN PATIENTEN
DISPOSITIF DE REFROIDISSEMENT CÉRÉBRAL POUR UN PATIENT DE CHIRURGIE À CRÂNE OUVERT

(30) Priority: 15.02.2012 KR 20120015558
(43) Date of publication of application: 24.12.2014
(73) Proprietor: Kyungpook National University Industry-Academic Cooperation Foundation, Daegu 702-701 (KR)
(72) Inventor: PARK, Jae Chan, Daegu 706-777 (KR); SUK, Kyoung Ho, Daegu 706-952 (KR); HAN, Hyung Soo, Daegu 706-935 (KR); RHEE, Dong Ick, Yangsan-si, Gyeongsangnam-do, 626-821 (KR)
(74) Representative: Dehns
(86) International application number: PCT/KR2013/000959
(87) International publication number: WO 2013/122351

(56) References cited:
- EP-A1- 1 514 529
- JP-A- 2007 209 523
- JP-A- 2011 083 315
- US-A1- 2002 077 682
- US-A1- 2002 077 682
- US-A1- 2002 198 579
- US-A1- 2010 198 319

## Description

### BACKGROUND

### 1. Field of the Invention

The present invention relates to a brain cooling device for a craniotomy patient, and more particularly, to a brain cooling device for a craniotomy patient that cools the brain serving as a region for surgery to prevent edema and damage to the patient's brain immediately after the craniotomy.

### 2. Discussion of Related Art

In general, in the case of various types of brain damage such as a cerebral infarction, a cerebral hemorrhage, head trauma, etc., the implementation of therapeutic hypothermia to drop brain temperature may help to alleviate cerebral edema, increased intracranial pressure, and the like due to a disease.

Here, to drop the brain temperature, the brain temperature may drop due to the decreased temperature of the whole body, however, effectively lowering the brain temperature by directly lowering the brain temperature is impossible because the brain is encompassed by the cranium and the scalp.

US 2002/077682, US 2002/198579 and EP 1514529 teach devices for cooling body tissue e.g. the brain.

The present invention, with an object to resolve the above-described problems, is directed to installing a brain cooling device through a region for surgery at the time of craniotomy, lowering the brain temperature during the period required after the surgery, and easily removing the brain cooling device inserted into the skull in a bed without further surgery once the therapeutic period has ended.

According to an aspect of the present disclosure, there is provided a brain cooling device for a craniotomy patient, comprising: a cooling portion which is buried in such a manner to come into contact with the brain or the dura mater; and one withdrawal tube, which is one end of the cooling portion exposed to the exterior, wherein the withdrawal tube is exposed to the exterior by penetrating through the scalp and the cranium.

According to another aspect of the present disclosure, there is provided a cooling portion which is buried in such a manner to come into contact with the brain or the dura mater; and an introduction tube and a discharge tube, which are two end portions of the cooling portion exposed to the exterior, wherein the introduction tube and the discharge tube are exposed to the exterior by penetrating through the scalp and the cranium.

According to another aspect of the present disclosure, the cooling portion may include soft tubes with a predetermined length.

According to another aspect of the present disclosure, the cooling portion may be configured to cover the upper part of the brain by forming multiple bent portions in a zigzag form.

According to another aspect of the present disclosure, a cooling material may be introduced or discharged through the withdrawal tube.

According to another aspect of the present disclosure, a cooling material may be introduced through the introduction tube and discharged through the withdrawal tube.

As described above, according to the brain cooling device for a craniotomy patient of the present invention, the cooling of brain can be effectively performed by installing a cooling portion that comes into direct contact with the brain or the dura mater, and using a cooling material which flows inside the cooling portion, and also the cooling portion can be easily removed in a bed.

### BRIEF DESCRIPTION OF THE DRAWINGS

FIG. 1 shows a brain cooling device for a craniotomy patient installed so that it can come into contact with the brain or the dura mater according to a first embodiment of the present invention;
FIG. 2 shows a schematic diagram of the internal structure of a cooling portion to be applied to the brain cooling device for a craniotomy patient according to the first embodiment of the present invention;
FIG. 3 shows a state of a fixed cranial bone flap after the completion of installing the brain cooling device for a craniotomy patient according to the first embodiment of the present invention;
FIG. 4 shows a state of a sutured scalp after the completion of installing the brain cooling device for a craniotomy patient according to the first embodiment of the present invention;
FIG. 5 shows an exemplary brain cooling device, not according to the invention, for a craniotomy patient installed so that it can come into contact with the brain or the dura mater;
FIG. 6 shows a schematic diagram of the internal structure of a cooling portion to be applied to the brain cooling device for a craniotomy patient not according to the invention;
FIG. 7 shows a state of a fixed cranial bone flap after the completion of installing an exemplary brain cooling device for a craniotomy patient not according to the invention; and
FIG. 8 shows a state of a sutured scalp after the completion of installing an exemplary brain cooling device for a craniotomy patient not according to the invention.

### DETAILED DESCRIPTION OF EXEMPLARY EMBODIMENTS

Exemplary embodiments of the present invention will be described in detail below with reference to the accompanying drawings.

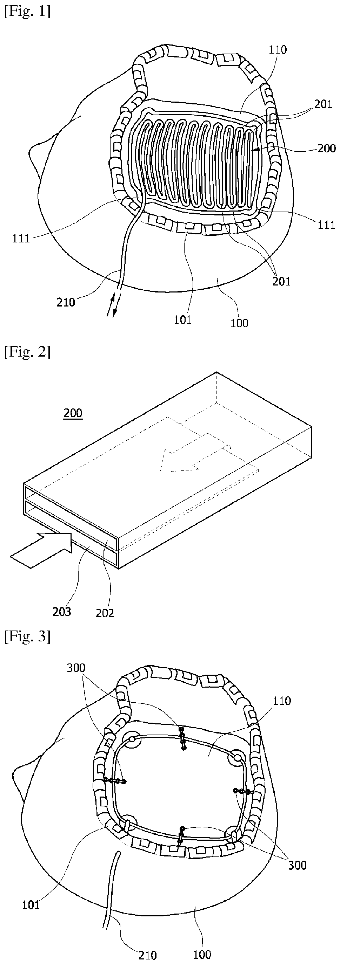

FIG. 1 shows a brain cooling device for a craniotomy patient installed so that it can come into contact with the brain or the dura mater according to a first embodiment of the present invention. As shown in FIG. 1, the brain cooling device for a craniotomy patient according to the first embodiment of the present invention includes a cooling portion 200, which is buried in such a manner to come into contact with the brain or the dura mater in a state in which a person's scalp 100 and cranium 110 are dissected and opened, and a withdrawal tube 210, which is exposed to the exterior by penetrating the scalp 100 and the cranium 110. In particular, the cooling portion 200 may be formed of a soft tube with a hollow center, i.e., in the form of a thin film formed of a material such as urethane or silicone.

Additionally, the cooling portion 200 is configured to form multiple bent portions in a zigzag form 201, and have a length sufficient to cover the upper portion of the brain. By installing it in this way, the area that comes in contact with the brain can be maximized, thereby more effectively performing the cooling activity.

Furthermore, the withdrawal tube 210 should be positioned through cranium perforations 111 formed during incision of the cranium 110, the penetration of the withdrawal tube 210 through the scalp 100 may be performed using a Trocar, and it is preferable that the withdrawal tube 210 be fixed around the scalp 100 using needles and threads for surgery.

Then, a cooling material is introduced and discharged through the withdrawal tube 210. Of course, the cooling material may be any material that enables a cooling activity by being subjected to heat exchange along with a physiological saline solution and nitrogen gas.

In addition, FIG. 2 shows a schematic diagram of the internal structure of a cooling portion to be applied to the brain cooling device for a craniotomy patient according to the first embodiment of the present invention. As shown in FIG. 2, if the cooling portion 200 is formed to be partitioned into an upper flow channel 202 and a lower flow channel 203, the cooling material may be introduced through one flow channel between the upper flow channel 202 and the lower flow channel 203, and at the same time, the cooling material may be discharged through the other flow channel, thereby minimizing the interference between introduction and discharge. As a result, the cooling efficiency can be improved.

FIG. 3 shows a state of a fixed cranial bone flap after the completion of installing the brain cooling device for a craniotomy patient according to the first embodiment of the present invention. As shown in FIG. 3, the withdrawal tube 210 may be exposed to the exterior by penetrating through the scalp using a trocar having a predetermined distance from the sewing portion 102. In particular, the withdrawal tube 210 may be fixed using needles and threads for surgery near the perforated portion of the scalp. Accordingly, the brain can be maintained at a low temperature by introducing or discharging a cooling material through the withdrawal tube 210.

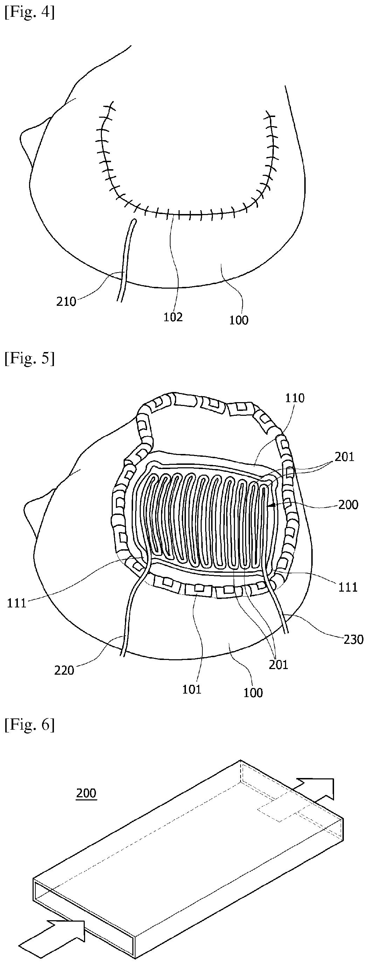

FIG. 4 shows a state of a sutured scalp after the completion of installing the brain cooling device for a craniotomy patient according to the first embodiment of the present invention. As shown in FIG. 4, when the scalp 100 is completely sealed by the sewing portion 102, the withdrawal tube 210 may remain exposed to the exterior. As such, the brain can be maintained at a low temperature by introducing or discharging a cooling material through the withdrawal tube 210. Later, the entire cooling portion 200 may be easily removed by cutting off the threads fixed to the withdrawal tube 210, and holding the withdrawal tube 210 with a hand and pulling it from the exterior of the scalp.

FIG. 5 shows a brain cooling device not according to the invention for a craniotomy patient installed so that it can come into contact with the brain or the dura mater. As shown in FIG. 5, the brain cooling device for a craniotomy patient not according to the invention has the same constitution as in the first embodiment described above in that the cooling portion 200 is buried in such a manner to come into contact with the brain or the dura mater in a state in which a person's scalp 100 and cranium 110 are dissected and opened. However, in the two end portions formed as an integral body extending from the cooling portion 200, the introduction tube 220 and the discharge tube 230 exposed to the exterior by penetrating through the scalp 100 and the cranium 110 are formed.

In particular, FIG. 6 shows a schematic diagram of the internal structure of a cooling portion to be applied to the brain cooling device for a craniotomy patient, not according to the invention. As shown in FIG. 6, although respective flow channels are formed inside the introduction tube 220 and the discharge tube 230, a cooling material is introduced through the introduction tube 220 and discharged through the discharge tube 230, and mutual interference between introduction and discharge at the time of introducing and discharging the cooling material can be prevented.

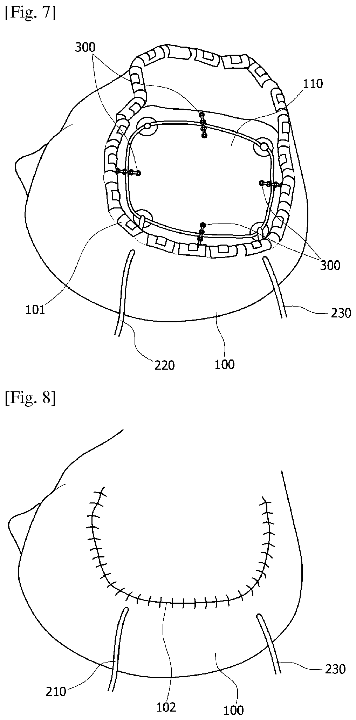

FIG. 7 shows a state of a sutured cranium after the completion of installing the brain cooling device for a craniotomy patient, not according to the invention. As shown in FIG. 7, if an already-open cranium 110 is closed and sealed tightly using an additionally provided binding member 300, the introduction tube 220 and the discharge tube 230 at the two ends that integrally extend from the cooling portion 200 are fixed to the perforations 111 of the cranium 110 and exposed to the exterior.

FIG. 8 shows a state of a sutured scalp after the completion of installing the brain cooling device for a craniotomy patient, not according to the invention. As shown in FIG. 8, the introduction tube 220 and the discharge tube 230 may be exposed to the exterior by penetrating through the scalp using a Trocar having a predetermined distance from the sewing portion 102. In particular, the introduction tube 220 and the discharge tube 230 are preferably fixed using needles and threads for surgery near the perforated portions of the scalp. By discharging a cooling material through the discharge tube 230 while simultaneously introducing the cooling material through the introduction tube 220, the cooling material may move at a faster flow rate, thereby further improving cooling performance. Later, the entire cooling portion 200 may be easily removed by cutting off any one of the threads fixed to introduction tube 220 and the discharge tube 230, and holding the other tube with a hand and pulling it from the exterior of the scalp.

The brain cooling device of the present invention, when applied during the implementation of therapeutic hypothermia to alleviate cerebral edema, increased intracranial pressure, etc., in the case of various types of brain damage such as a cerebral infarction, a cerebral hemorrhage, head trauma, etc., helps to effectively lower brain temperature, and a cooling portion can be easily removed in a bed. The brain cooling device of the present invention may be used in the field of medical devices for improving the efficiency of the therapeutic hypothermia.

## Claims

1. A brain cooling device for a craniotomy patient, comprising:
a cooling portion (200) which is buried in such a manner to come into contact with the brain or the dura mater; and
a tube portion (210) formed in one end portion of the cooling portion and exposed to the exterior by penetrating through the scalp (100) and the cranium (110),
wherein the cooling portion is formed to be partitioned into an upper flow channel (202) and a lower flow channel (203) located between the upper flow channel and the brain or the dura mater, and a cooling material is introduced through one of the upper flow channel and the lower flow channel, and at the same time, the cooling material is discharged through the other flow channel;
**characterised in that** the cooling portion comprises a soft tube with a predetermined length; and the cooling portion is configured to cover a part of the brain by forming multiple bent portions in a zigzag form (201).

2. The brain cooling device according to claim 1, wherein the cooling material is introduced and discharged through the tube portion (210).

## Patentansprüche

1. Hirnkühlungsvorrichtung für einen Kraniotomiepatienten, umfassend:
einen Kühlabschnitt (200), der auf eine solche Weise eingepflanzt ist, dass er mit dem Hirn oder der Dura mater in Berührung kommt; und
einen Rohrabschnitt (210), der in einem Endabschnitt des Kühlabschnitts gebildet ist und gegenüber dem Außenbereich freiliegt, indem er die Kopfhaut (100) und den Schädel (110) durchdringt,
wobei der Kühlabschnitt gebildet ist, um in einen oberen Strömungskanal (202) und einen unteren Strömungskanal (203), der sich zwischen dem unteren Strömungskanal und dem Hirn oder der Dura mater befindet, unterteilt zu sein, und ein Kühlmaterial durch einen von dem oberen Strömungskanal und dem unteren Strömungskanal eingeleitet wird und das Kühlmaterial gleichzeitig durch den anderen Strömungskanal abgegeben wird;
**dadurch gekennzeichnet, dass**
der Kühlabschnitt ein Weichstoffrohr mit einer vorbestimmten Länge umfasst; und
der Kühlabschnitt konfiguriert ist, um einen Teil des Hirns durch Bilden von mehreren gebogenen Abschnitten in einer Zickzack-Form (201) zu bedecken.

2. Hirnkühlvorrichtung nach Anspruch 1, wobei das Kühlmaterial durch den Rohrabschnitt (210) eingeleitet und abgegeben wird.

## Revendications

1. Dispositif de refroidissement cérébral pour un patient souffrant de craniotomie, comprenant :
une partie de refroidissement (200) qui est enterrée de manière à entrer en contact avec le cerveau ou la dure-mère ; et
une partie de tube (210) formée dans une partie d'extrémité de la partie de refroidissement et exposée à l'extérieur par la pénétration à travers le cuir chevelu (100) et le crâne (110),
dans lequel la partie de refroidissement est formée pour être divisée en un canal d'écoulement supérieur (202) et un canal d'écoulement inférieur (203) situé entre le canal d'écoulement supérieur et le cerveau ou la dure-mère,
et un matériau de refroidissement est introduit à travers l'un du canal d'écoulement supérieur et du canal d'écoulement inférieur, et en même temps, le matériau de refroidissement est déchargé à travers l'autre canal d'écoulement ;
**caractérisé en ce que**
la partie de refroidissement comprend un tube souple ayant une longueur prédéterminée ; et
la partie de refroidissement est configurée pour recouvrir une partie du cerveau par la formation de plusieurs parties courbées en forme de zigzag (201).

2. Dispositif de refroidissement cérébral selon la revendication 1, dans lequel le matériau de refroidissement est introduit et déchargé à travers la partie de tube (210).
